# EUROPÄISCHE PATENTSCHRIFT

(11) **EP 1 166 772 B1**
(45) Veröffentlichungstag und Bekanntmachung des Hinweises auf die Patenterteilung: **28.12.2005**
(21) Anmeldenummer: 00112547.5
(22) Anmeldetag: 14.06.2000
(51) Int. Cl.: A61K 7/50

(54) **Kosmetische Reinigungsemulsion mit hohem Ölgehalt**
Cosmetic cleansing composition with a high oil concentration
Composition cosmetique de nettoyage à haute concentration en huile

(43) Veröffentlichungstag der Anmeldung: 02.01.2002
(73) Patentinhaber: Beiersdorf AG, 20253 Hamburg (DE)
(72) Erfinder: Ruppert, Stephan, Dr., 20253 Hamburg (DE); Kohut, Michaela, 20257 Hamburg (DE)

(56) Entgegenhaltungen:
- EP-A- 0 739 619
- EP-A- 0 870 495
- DE-A- 19 703 087
- FR-A- 2 780 278
- "International Cosmetic Ingredient Dictionary and Handbook" 1997 , THE COSMETIC,TOILETRY, AND FRAGRANCE ASSOCIATION , WASHINGTON, USA XP002149876 * Seite 26 *
- INTERNATIONAL JOURNAL OF PHARMACEUTICS, Bd. 258, 2003, Seiten 179-191, POLYACID MICROGELS - CARBOPOL 934 - SURFACTANT INTERACTIONS IN AQUEOUS MEDIA, PART II IONIC SURFACTANTS
- 1994, A. DOMSCH , DIE KOSMETISCHEN PRÄPARATE, BD III, 4. AUFLAGE, S. 106-114

## Beschreibung

Die vorliegende Erfindung betrifft kosmetische Reinigungsemulsionen, insbesondere solche, die keinen Emulgator im herkömmlichen Sinn enthalten.

Die Produktion von kosmetischen Reinigungsmitteln zeigt seit Jahren eine steigende Tendenz. Dies ist vor allem auf das zunehmende Gesundheitsbewußtsein und Hygienebedürfnis der Verbraucher zurückzuführen.

Reinigung bedeutet das Entfernen von (Umwelt-) Schmutz und bewirkt damit eine Erhöhung des psychischen und physischen Wohlbefindens. Die Reinigung der Oberfläche von Haut und Haaren ist ein sehr komplexer, von vielen Parametern abhängiger Vorgang. Zum einen sollen von außen kommende Substanzen wie beispielsweise Kohlenwasserstoffe oder anorganische Pigmente aus unterschiedlichsten Umfeldern sowie Rückstände von Kosmetika oder auch unerwünschte Mikroorganismen möglichst vollständig entfemt werden. Zum anderen sind körpereigene Ausscheidungen wie Schweiß, Sebum, Haut- und Haarschuppen ohne tiefgreifende Eingriffe in das physiologische Gleichgewicht abzuwaschen.

Die Forderungen an die Eigenschaften kosmetischer Reinigungspräparate haben sich in den letzten Jahren stark gewandelt. Früher standen Effekte wie Reinigen und Schäumen im Vordergrund der Verbraucherwünsche. Zur Zeit sind die ökologischen, ökonomischen und insbesondere dermatologischen Eigenschaften der Produkte vorrangig, obwohl das Schaumvermögen nach wie vor eine entscheidende Rolle spielt, beispielsweise als Indikator um Restmengen von Tensiden nach der Reinigung von Haut und Haaren zu entfernen oder um Überdosierungen bei der Anwedung zu vermeiden. Allerdings steht bei kosmetischen Produkten - im Gegensatz zu den meisten technischen Reinigungsmitteln - die Haut- und Schleimhautverträglichkeit absolut im Vordergrund; die Produkte sollen "mild" sein

Kosmetische oder dermatologische Reinigungspräparate sind sogenannte "rinse off" Präparate, welche nach der Anwendung von der Haut abgespült werden. Sie werden in aller Regel in Form eines Schaums mit Wasser auf die zu reinigenden Körperpartien aufgetragen Basis aller kosmetischen oder dermatologischen Reinigungspräparate sind waschaktive Tenside. Tenside sind amphiphile Stoffe, die organische, unpolare Substanzen in Wasser lösen können. Sie zeichnen sich durch ein ambivalentes Verhalten gegenüber Wasser und Lipiden aus: Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfernung und -lösung, ein leichtes Abspülen und - je nach Wunsch - auch für Schaumregulierung. Damit ist die Grundlage für die Schmutzentfernung lipidhaltiger Verschmutzungen gegeben.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren öder neutralen Milieu negativ geladene organische Ionen. Kationische Tenside sind beinahe ausschließlich durch das Vorhandensein einer quartemären Ammoniumgruppe gekennzeichnet. In wäßriger Lösung bilden sie im sauren oder neutralen Milieu positiv geladene organische lonen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

RNH₂⁺CH₂CH₂COOH X⁻ (bei pH=2)

X⁻ = beliebiges Anion, z.B. Cl⁻

RNH₂⁺CH₂CH₂COO⁻ (bei pH=7)

RNHCH₂CH₂COO⁻ B⁺ (bei pH=12)

B⁺ = beliebiges Kation, z.B. Na⁺

Typisch für nicht-ionische Tenside sind Polyether-Ketten. Nicht-ionische Tenside bilden in waßngem Medium keine Ionen.

Die waschaktiven Tenside in kosmetischen und dermatologischen Reinigungsmitteln unterliegen einer sehr kritischen Beurteilung bezüglich ihres dermatologischen und ökologischen Verhaltens. Letzteres ist insbesondere deswegen von Bedeutung, da sie in erheblicher Menge angewendet werden und nach Gebrauch bestimmungsgemäß ins Abwasser gelangen.

Ausgehend von der bereits beschriebenen zentralen Bedeutung der waschaktiven Tenside für den Reinigungsvorgang ist ihr Verhalten auf der Humanhaut von größter Bedeutung

Bereits bei einer Reinigung der Haut mit Hilfe von Wasser - ohne Zusatz von Tensiden - kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut.

Es ist verständlich, daß waschaktive Tenside, die Haut und Haar von fettigen und wasserlöslichen Schmutzbestandteilen reinigen sollen, auch eine entfettende Wirkung auf die normalen Hautlipide haben. Bei jeder Hautreinigung werden in unterschiedlichem Maß auch interkomeozytäre Lipide und Sebumbestandteile entfernt. Das bedeutet, daß der natürliche Wasser-Lipid-Mantel der Haut bei jedem Waschvorgang mehr oder weniger gestört wird. Dies kann besonders bei extremer Entfettung zu einer kurzzeitigen Veränderung der Barrierefunktion der Haut führen, wobei selbstverständlich auch der jeweilige Zustand der behandelten Hautregion auf die dargestellten Veränderungen von erheblichem Einfluß ist. Beispielsweise kann die Hautdicke, die Anzahl der Talg- und Schweißdrusen sowie die damit verbundene Empfindlichkeit erheblich variieren.

Grundsätzlich gilt dementsprechend als Forderung an waschaktive Tenside, daß sie biologisch möglichst inaktiv sind, um unerwünschte Nebenwirkungen zu vermeiden. Sie sollen ihre reinigende Wirkung bei optimaler Milde, bester Hautverträglichkeit und geringer Entfettung entfalten.

Es hat daneben aber auch nicht an Versuchen gefehlt, geeignete Reinigungszubereitungen zu finden, welche die Haut bei guter Reinigungsleistung gleichzeitig regenerieren bzw. "rückfetten". Allerdings bleibt die erzielte Leistung häufig hinter der erwarteten zurück, so daß der Anwender in aller Regel auf separate Pflegeprodukte zurückgreifen muß, welche nach der Reinigung auf die Haut aufgetragen werden und auf dieser verbleiben (sogenannte "leave-on" Produkte).

Bedingt durch die zunehmende Waschfrequenz beim Verbraucher steigt allerdings nach wie vor der Wunsch nach milden, verträglichen und dabei möglichst regenerierenden Zubereitungen.

In der Regel sind kosmetische oder dermatologische Reinigungszubereitungen sehr gut auf ein angenommenes Anwendungsspektrum zugeschnitten, da für eine definierte, milde Reinigungswirkung insbesondere auch die je nach Anwendung unterschiedlichen mechanischen Parameter - wie beispielsweise der Zeitfaktor - von erheblicher Bedeutung sind: Dies wird z. B. deutlich, wenn man sich die unterschiedlichen Anwendungs- (Kontakt-) Zeiten eines Schaumbades im Vergleich zum kurzzeitigen Händewaschen vor Augen führt.

Kosmetische Reinigungsmittel enthalten meist Mischungen von Tensiden verschiedener Art. Die Auswahl orientiert sich in erster Linie an der Hautverträglichkeit und der gewunschten kosmetischen Leistung der Tenside. Daneben spielen Schaumvermögen, Formulierbarkeit und ein günstiges Leistungs-/Kostenverhältnis eine wesentliche Rolle.

Flüssige Seifen oder Waschlotionen werden nicht nur zur Reinigung der Hände, sondern im Regelfall auch für den ganzen Körper, einschließlich des Gesichts, verwendet. Sie eignen sich dementsprechend auch zur Anwendung als Duschzubereitung. Bei der Entwicklung dieser Produkte stehen die dermatologischen Anforderungen im Vordergrund, da die Haut in intensiven Kontakt mit der konzentrierten Tensidlösung kommt. Auf die Auswahl milder Tenside in niedriger Konzentration wird daher besonderer Wert gelegt. Weitere Kriterien sind ferner ein gutes Schaumvermögen sowie ein angenehmer, erfrischender Duft und die gleichzeitige Pflege der Haut. Waschlotionen und insbesondere Duschbäder haben in der Regel Viskositäten von etwa 3.000 bis 10.000 mPa·s, welche einerseits eine gute Verteilbarkeit des Produktes mit schnellem Anschäumen erlauben, dabei andererseits aber hoch genug sein sollen, um eine einwandfreie Anwendung per Hand oder Waschlappen zu ermöglichen.

Flüssige Seifen oder Waschlotionen zeichnen sich im allgemeinen durch einen mehr oder weniger hohen Wassergehalt aus, entfalten aber in der Regel keine nennenswerte Pflegewirkung da sie nur einen geringen Ölgehalt aufweisen.

Eine relativ neue technische Entwicklung sind tensidhaltige Duschzubereitungen mit hohem Ölgehalt. Die Deutsche Offenlegungsschrift 44 24 210 beschreibt in diesem Zusammenhang kosmetische oder dermatologische Duschzubereitungen mit einem Tensidgehalt von höchstens 55 Gew.-% und einem Ölgehalt von mehr als 45 Gew.-%, wobei die Zubereitungen im wesentlichen wasserfrei sind. Aufgrund des hohen Ölgehalts wirken diese Zubereitungen regenerierend in bezug auf den allgemeinen Hautzustand. Sie haben dabei gleichzeitig eine gute Schaumentwicklung und eine hohe Reinigungskraft.

Ferner beschreibt WO 96/17591 schäumende flüssige Hautreinigungszusammensetzungen, welche die folgenden Substanzen enthalten: 5 bis 30 Gew.-% eines feuchtigkeitsspendenden Wirkstoffs, welcher einen Vaughan Solubility Parameter (VSP) von 5 bis 10 aufweist, 0,3 bis 5 Gew.-% eines in Wasser dispergierbaren gelformenden Polymers, 5 bis 30 Gew.-% einer synthetischen oberflächenaktiven Substanz, 0 bis 15 Gew.-% einer C₈ bis C₁₄ Fettsäureseife und Wasser, wobei die Zubereitungen einen Lipid Deposition Value (LDV) von mindestens 5 bis 1000 aufweisen und worin die synthetische oberflächenaktiven Substanz und die Seife einen gemeinsamen CMC Gleichgewichtsoberflächenspannungswert von 15 bis 50 haben. Allerdings konnte diese Schrift nicht den Weg zur vorliegenden Erfindung weisen.

Die EP 0 870 495-A2 beschreibt die Verwendung von Mischungen, die bezogen auf das Gesamtgewicht der Zubereitungen 1 bis 50 Gew.-% eines oder mehrerer Tenside und 5 bis 60 Gew.-% Wasser und 25 bis 90 Gew.-% einer oder mehrerer Ölkomponenten enthalten, als kosmetische und/oder dermatologische Reinigungszubereitungen.

Der Stand der Technik kennt zur Reinigung und gleichzeitigen Pflege der Haut ferner auch Reinigungsprodukte auf Emulsionsbasis. Diese werden in der Art formuliert, daß die Emulsion mit Emulgatoren stabilisiert und anschließend ein Tensidsystem angepaßt wird. Auch Emulgatoren haben eine amphiphile Struktur, sind also den Tensiden von der Struktur her vergleichbar. Emulgatoren ermöglichen oder erleichtern die gleichmäßige Verteilung zweier oder mehrerer miteinander nicht mischbarer Phasen und verhindern gleichzeitig deren Entmischung. Da Emulsionen durch die Zugabe von Tensiden im allgemeinen zerstört werden, ist die Wahl des Tensidsystems stark eingeschränkt, und den erhaltenen Reinigungszubereitungen liegen teure und komplizierte Rezepturen zugrunde.

### Was unterscheidet nun waschaktive Tenside von Emulgatoren?

Ende der vierziger Jahre wurde ein System entwickelt, das die Auswahl von Emulgatoren erleichtern sollte. Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen öllösliche, hydrophobe Emulgatoren, Zahlen über 11 wasserlösliche, hydrophile. Der HLB-Wert sagt etwas über das Gleichgewicht der Größe und Stärke der hydrophilen und der lipophilen Gruppen eines Emulgators aus. Aus diesen Überlegungen läßt sich ableiten, daß auch die Wirksamkeit eines Emulgators durch seinen HLB-Wert charakterisiert werden kann. Die folgende Aufstellung zeigt den Zusammenhang zwischen HLB-Wert und möglichem Anwendungsgebiet:

| **HLB-Wert** | **Anwendungsgebiet** |
|---|---|
| 0 bis 3 | Entschäumer |
| 3 bis 8 | W/O-Emulgator |
| 7 bis 9 | Netzmittel |
| 8 bis 18 | O/W-Emulgator |
| 12 bis 18 | Lösungsvermittler |

Der HLB-Wert eines Emulgators läßt sich auch aus Inkrementen zusammensetzen, wobei die HLB-Inkremente für die verschiedenen hydrophilen und hydrophoben Gruppen, aus denen sich ein Molekül zusammensetzt, Tabellenwerken entnommen werden können. Auf diese Weise lassen sich im Prinzip auch für waschaktive Tenside HLB-Werte ermitteln, obwohl das HLB-System ursprünglich nur für Emulgatoren konzipiert worden ist. Es zeigt sich, daß waschaktive Substanzen in der Regel HLB-Werte aufweisen, die deutlich größer als 20 sind.

Aufgabe der vorliegenden Erfindung war es, Reinigungszubereitungen auf der Grundlage von Emulsionen zur Verfügung zu stellen, welche den Nachteilen des Standes der Technik Abhilfe schaffen und denen dementsprechend einfache und kostengünstige Rezepturen zugrunde liegen. Die Zubereitungen sollten zudem eine hohe Pflegewirkung besitzen, ohne daß die reinigende Wirkung dahinter zurücksteht.

Die vorliegende Erfindung betrifft ferner waschaktive haarkosmetische Zubereitungen, im allgemeinen als Shampoos bezeichnet. Insbesondere betrifft die vorliegende Erfindung haarkosmetische Wirkstoffkombinationen und Zubereitungen zur Pflege des Haars und der Kopfhaut. Dem Stand der Technik mangelt es an Shampooformulierungen, welche geschädigtem Haar in befriedigender Weise Pflege zukommen lassen. Aufgabe war daher, auch diesen Nachteilen des Stands der Technik Abhilfe zu schaffen.

Es hat sich überraschend gezeigt, und darin liegt die Lösung dieser Aufgaben, daß kosmetische oder dermatologische Reinigungsemulsionen, dadurch gekennzeichnet, dass sie bezogen auf das Gesamtgewicht der Zubereitungen
■ 1 bis 30 Gew.-% eines oder mehrerer waschaktiver Tenside, gewählt aus der Gruppe der Tenside, welchen einen HLB-Wert von mehr als 35 haben,
■ 35 bis 50 Gew.-% einer oder mehrerer Ölkomponenten,
■ 0,2 bis 5 Gew.-% eines oder mehrerer Polyacrylate, gewählt aus der Gruppe, welche gebildet wird aus anionischen Homo- und/oder Copolymeren der Acrylsäure und/oder alkylierten Acrylsäurederivaten sowie deren Estern und
■ 5 bis 60 Gew.-% Wasser
enthalten,
den Nachteilen des Standes der Technik abhelfen.

Den kosmetischen und/oder dermatologischen Reinigungsemulsionen im Sinn der vorliegenden Erfindung liegen einfache und kostengünstige Rezepturen zugrunde. Sie haben gleichzeitig eine gute Schaumentwicklung und eine hohe Reinigungskraft. Aufgrund des hohen Ölgehalts wirken diese Zubereitungen regenerierend in bezug auf den allgemeinen Hautzustand, vermindern das Trockenheitsgefühl der Haut und machen die Haut geschmeidig.

Die Reinigungsemulsionen enthalten ein oder mehrere erfindungsgemäße waschaktive anionische, kationische, amphotere und/oder nicht-ionische Tenside, welche einen HLB-Wert von mehr als 35 haben.

Ganz besonders vorteilhafte waschaktive anionische Tenside im Sinne der vorliegenden Erfindung sind
Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,
Sulfonsäuren und deren Salze, wie
■ Acyl-isethionate, z.B. Natrium-/ Ammoniumcocoyl-isethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Ganz besonders vorteilhafte waschaktive kationische Tenside im Sinne der vorliegenden Erfindung sind quartemäre Tenside. Quatemäre Tenside enthalten mindestens ein N-Atom, das mit 4 Alkyl- oder Arylgruppen kovalent verbunden ist. Vorteilhaft sind Benzalkoniumchlorid, Alkylbetain, Alkylamidopropylbetain und Alkyl-amtdopropylhydroxysultain.

Ganz besonders vorteilhafte waschaktive amphotere Tenside im Sinne der vorliegenden Erfindung sind
■ Acyl-/dialkylethylendiamine, beispielsweise Natriumacylamphoacetat, Dinatriumacylämphodipropionat, Dinatriumalkylamphodiacetat, Natriumacylamphohydroxypropylsulfonat, Dinatriumacylamphodiacetat und Natriumacylamphopropionat,

Ganz besonders vorteilhafte waschaktive nicht-ionische Tenside im Sinne der vorliegenden Erfindung sind
■ Älkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat,
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole und
■ ethoxylierte Amine.

Weitere geeignete nicht-ionische Tenside im Sinne der vorliegenden Erfindung sind ferner Aminoxide, wie Cocoamidopropylaminoxid.

Es ist vorteilhaft im Sinn der vorliegenden Erfindung, wenn der Gehalt an einem oder mehreren waschaktiven Tensiden in der kosmetischen oder dermatologischen Reinigungsemulsion aus dem Bereich von 5 bis 25 Gew.-%, ganz besonders vorteilhaft von 10 bis 20 Gew.-% gewählt wird, jeweils bezogen auf das Gesamtgewicht der Zubereitungen.

Die Ölphase der kosmetischen oder dermatologischen Reinigungsemulsionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretrigtyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natünichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und lsotridecylisononanoat, aus Cyclomethicon und 2-Ethylhexylisostearat.

Die Ölphase wird ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur 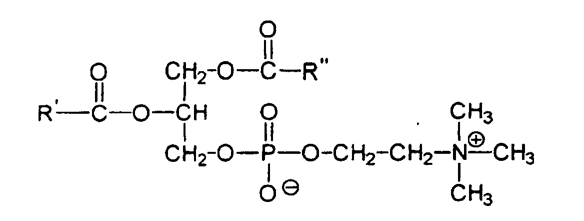 auszeichnen, wobei R' und R" typischerweise unverzweigte aliphatische Reste mit 15 oder 17 Kohlenstoffatomen und bis zu 4 cis-Doppelbindungen darstellen.

Erfindungsgemäß vorteilhafte Polyacrylate sind Polymere der Acrylsäure, insbesondere solche, die aus der Gruppe der sogenannten Carbomere oder Carbopole (Carbopol® ist eigentlich eine eingetragene Marke der B. F. Goodrich Company) gewählt werden. Polyacrylate sind Verbindungen der allgemeinen Strukturformel 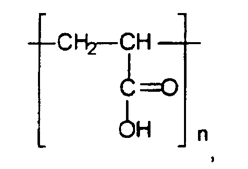 deren Molgewicht zwischen ca. 400 000 und mehr als 4 000 000 betragen kann. In die Gruppe der Polyacrylate gehören ferner Acrylat-Alkylacrylat-Copolymere, beispielsweise solche, die sich durch die folgende Struktur auszeichnen:

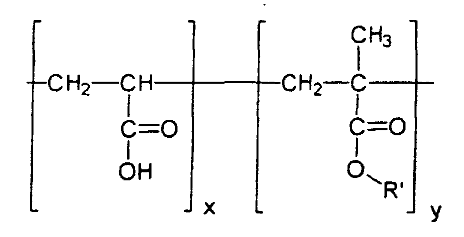

Darin stellen R' einen langkettigen Alkylrest und x und y Zahlen dar, welche den jeweiligen stöchiometrischen Anteil der jeweiligen Comonomere symbolisieren. Auch diese Polyacrylate sind vorteilhaft im Sinne der vorliegenden Erfindung.

Vorteilhafte Carbopole sind beispielsweise die Typen 907, 910, 934, 940, 941, 951, 954, 980, 981, 1342, 1382, 2984 und 5984 oder auch die Typen ETD (Easy-to-disperse) 2001, 2020, 2050, wobei diese Verbindungen einzeln oder in beliebigen Kombinationen untereinander vorliegen können.

Besonders bevorzugt sind Carbopol 981, 1382 und ETD 2020 (sowohl einzeln als auch in Kombination).

Ferner vorteilhaft im Sinne der vorliegenden Erfindung sind die den Acrylat-Alkylacrylat-Copolymeren vergleichbaren Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die unter den Handelsbezeichnungen Pemulen TR1 und Pemulen TR2 bei der B. F. Goodrich Company erhältlichen.

Es ist vorteilhaft im Sinn der vorliegenden Erfindung, wenn der Gehalt an einem oder mehreren Polyacrylaten in der kosmetischen oder dermatologischen Reinigungsemulsion aus dem Bereich von 0,5 bis 2 Gew.-%, ganz besonders vorteilhaft von 0,7 bis 1,5 Gew.-% gewählt wird, jeweils bezogen auf das Gesamtgewicht der Zubereitungen

Kosmetische Zubereitungen, die kosmetische Reinigungszubereitungen für die Haut darstellen, können in flüssiger oder fester Form vorliegen.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipröpionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin. Ferulasaure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Emulsionen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Die folgenden Beispiele, in welchen Waschpräparate zur Haar- und Körperpflege beschrieben werden, sollen die erfindungsgemäßen Zusammensetzungen erläutern, ohne daß aber beabsichtigt ist, die Erfindung auf diese Beispiele zu beschränken. Die Zahlenwerte in den Beispielen bedeuten Gewichtsprozente, bezogen auf das Gesamtgewicht der jeweiligen Zubereitungen.

### Beispiele:

| **Phase** | **Bestandteil** | **Menge** |
|---|---|---|
| **A** | Natriumlaurethsulfat (25%-ige Lösung in Wasser) | 44,00 |
| **B** | Mineral Oil (Paraffinum Liquidum) | 20,00 |
| | Acrylates/C10-30 Alkyl Acrylate Crosspolymer | 1,00 |
| **C** | Glycine Soja + (Ricinus Communis) + Propyl Gallate | 20,00 |
| | Sodium Hydroxide, Antioxidantien, Konservierung, Parfum | q.s. |
| | Water (Aqua) | ad 100 |

| **Phase** | **Bestandteil** | **Menge** |
|---|---|---|
| **A** | Natriumlaurethsulfat (25%-ige Lösung in Wasser) | 44,00 |
| **B** | Mineral Oil (Paraffinum Liquidum) | 20,00 |
| | Acrylates/C10-30 Alkyl Acrylate Crosspolymer | 0,90 |
| **C** | Glycine Soja + (Ricinus Communis) + Propyl Gallate | 20,00 |
| | Sodium Hydroxide, Antioxidantien, Konservierung, Parfum | q.s. |
| | Water (Aqua) | ad 100 |

| **Phase** | **Bestandteil** | **Menge** |
|---|---|---|
| **A** | Natriumlaurethsulfat (25%-ige Lösung in Wasser) | 44,00 |
| **B** | Mineral Oil (Paraffinum Liquidum) | 20,00 |
| | Acrylates/C10-30 Alkyl Acrylate Crosspolymer | 1,00 |
| **C** | Glycine Soja + (Ricinus Communis) + Propyl Gallate | 15,00 |
| | Sodium Hydroxide, Antioxidantien, Konservierung-Parfum | q.s. |
| | Water (Aqua) | ad 100 |

| **Phase** | **Bestandteil** | **Menge** |
|---|---|---|
| **A** | Natriumlaurethsuifat (25%-ige Lösung in Wasser) | 44,00 |
| **B** | Mineral Oil (Paraffinum Liquidum) | 18,00 |
| | Acrylates/C10-30 Alkyl Acrylate Crosspolymer | 1,20 |
| **C** | Glycine Soja + (Ricinus Communis) + Propyl Gallate | 17,00 |
| | Sodium Hydroxide, Antioxidantien, Konservierung, Parfum | q.s. |
| | Water (Aqua) | ad 100 |

| **Phase** | **Bestandteil** | **Menge** |
|---|---|---|
| **A** | Natriumlaurethsulfat (25%-ige Lösung in Wasser) | 44,00 |
| **B** | Mineral Oil (Paraffinum Liquidum) | 25,00 |
| | Acrytates/C10-30 Alkyl Acrylate Crosspolymer | 0,80 |
| **C** | Glycine Soja + (Ricinus Communis) + Propyl Gallate | 25,00 |
| | Sodium Hydroxide, Antioxidantien, Konservierung, Parfum | q.s. |
| | Water (Aqua) | ad 100 |

| **Phase** | **Bestandteil** | **Menge** |
|---|---|---|
| **A** | Natriumlaurethsulfat (25%-ige Lösung in Wasser) | 24,00 |
| **B** | Mineral Oil (Paraffinum Liquidum) | 20,00 |
| | Acrylates/C10-30 Alkyl Acrylate Crosspolymer | 1,00 |
| **C** | Glycine Soja + (Ricinus Communis) + Propyl Gallate | 20,00 |
| | Sodium Hydroxide, Antioxidantien, Konservierung, Parfum | q.s. |
| | Water (Aqua) | ad 100 |

| **Phase** | **Bestandteil** | **Menge** |
|---|---|---|
| **A** | Natriumlaurethsulfat (25%-ige Lösung in Wasser) | 54,00 |
| **B** | Mineral Oil (Paraffinum Liquidum) | 20,00 |
| | Acrylates/C10-30 Alkyl Acrylate Crosspolymer | 1,00 |
| **C** | Glycine Soja + (Ricinus Communis) + Propyl Gallate | 20,00 |
| | Sodium Hydroxide, Antioxidantien, Konservierung, Parfum | q.s. |
| | Water (Aqua) | ad 100 |

| **Phase** | **Bestandteil** | **Menge** |
|---|---|---|
| **A** | Natriumlaurethsulfat (25%-ige Lösung in Wasser) | 40,00 |
| | Cocamidopropyl Betaine, Water(Aqua) | 8,80 |
| **B** | Mineral Oil (Paraffinum Liquidum) | 20,00 |
| | Acrylates/C90-30 Alkyl Acrylate Crosspolymer | 0,75 |
| **C** | Glycine Soja + (Ricinus Communis) + Propyl Gallate | 20,00 |
| | Sodium Hydroxide, Antioxidantien, Konservierung, Parfum | q.s. |
| | Water (Aqua) | ad 100 |

### Herstellanweisung:

**1.** Bestandteile der Phase (B) mischen bis der Verdicker gleichmäßig suspendiert ist.
**2.** Phase (B) mit Phase (C) mischen; andere lipophile Bestandteile zugeben.
**3.** Bestandteile der Phase (A) mit Wasser mischen; andere hydrophile Bestandteile zugeben.
**4.** Phase (A) zu Phase (B)+(C) geben; homogenisieren.

## Patentansprüche

1. Kosmetische oder dermatologische Reinigungsemulsionen, **dadurch gekennzeichnet, dass** sie bezogen auf das Gesamtgewicht der Zubereitungen
■ 1 bis 30 Gew.-% eines oder mehrerer waschaktiver Tenside, gewählt aus der Gruppe der Tenside, welchen einen HLB-Wert von mehr als 35 haben,
■ 35 bis 50 Gew.-% einer oder mehrerer Ölkomponenten,
■ 0,2 bis 5 Gew.-% eines oder mehrerer Polyacrylate, gewählt aus der Gruppe, welche gebildet wird aus anionischen Homo- und/oder Copolymeren der Acrylsäure und/oder alkylierten Acrylsäurederivaten sowie deren Estern und
■ 5 bis 60 Gew.-% Wasser
enthalten.

2. Kosmetische oder dermatologische Reinigungsemulsionen nach Anspruch 1, **dadurch gekennzeichnet, daß** zusätzlich weitere kosmetische oder pharmazeutische Hilfs-, Zusatz- und/oder Wirkstoffe enthalten sind, die den Wassergehalt der Formulierungen vermindern.

3. Kosmetische oder dermatologische Reinigungsemulsionen nach einem der vorhergehenden Ansprüche, **dadurch gekennzeichnet, dass** das oder die Polyacrylate aus der Gruppe der Polymere der Acrylsäure und/oder deren Ester und/oder der Gruppe der Acrylat-Alkylacrylat-Copolymere gewählt werden.

4. Kosmetische oder dermatologische Reinigungsemulsionen nach einem der vorhergehenden Ansprüche, **dadurch gekennzeichnet, dass** das Tensid Natrium-Laurethsulfat ist.

5. Kosmetische oder dermatologische Reinigungsemulsionen, nach einem der vorhergehenden Ansprüche **dadurch gekennzeichnet, dass** sie bezogen auf das Gesamtgewicht der Zubereitungen
■ 5 bis 20 Gew.-% eines oder mehrerer waschaktiver Tenside, gewählt aus der Gruppe der Tenside, welchen einen HLB-Wert von mehr als 35 haben,
■ 35 bis 50 Gew.-% einer oder mehrerer Ölkomponenten,
■ 0,5 bis 2 Gew.-% eines oder mehrerer Polyacrylate, gewählt aus der Gruppe, welche gebildet wird aus anionischen Homo- und/oder Copolymeren der Acrylsäure und/oder alkylierten Acrylsäurederivaten sowie deren Estern und
■ 5 bis 60 Gew.-% Wasser
enthalten.

## Claims

1. Cosmetic or dermatological cleansing emulsions **characterized in that**, based on the total weight of the preparations, they comprise
■ 1 to 30% by weight of one or more washing-active surfactants chosen from the group of surfactants which have an HLB value of more than 35,
■ 35 to 50% by weight of one or more oil components,
■ 0.2 to 5% by weight of one or more polyacrylates chosen from the group which is formed from anionic homopolymers and/or copolymers of acrylic acid and/or alkylated acrylic acid derivatives, and esters thereof and
■ 5 to 60% by weight of water.

2. Cosmetic or dermatological cleansing emulsions according to Claim 1, **characterized in that** in addition further cosmetic or pharmaceutical auxiliaries, additives and/or active ingredients are present which reduce the water content of the formulations.

3. Cosmetic or dermatological cleansing emulsions according to one of the preceding claims, **characterized in that** the polyacrylate or polyacrylates are chosen from the group of polymers of acrylic acid and/or esters thereof and/or the group of acrylate/alkyl acrylate copolymers.

4. Cosmetic or dermatological cleansing emulsions according to one of the preceding claims, **characterized in that** the surfactant is sodium laureth sulphate.

5. Cosmetic or dermatological cleansing emulsions, **characterized in that**, based on the total weight of the preparation according to one of the preceding claims, they comprise
■ 5 to 20% by weight of one or more washing-active surfactants chosen from the group of surfactants which have an HLB value of more than 35,
■ 35 to 50% by weight of one or more oil components,
■ 0.5 to 2% by weight of one or more polyacrylates chosen from the group which is formed from anionic homopolymers and/or copolymers or acrylic acid and/or alkylated acrylic acid derivatives, and esters thereof and
■ 5 to 60% by weight of water.

## Revendications

1. Emulsions de nettoyage cosmétiques ou dermatologiques, **caractérisées en ce qu'**elles contiennent, par rapport au poids total des compositions
- 1 à 30% en poids d'une ou de plusieurs substances tensioactives détergentes choisies dans le groupe des substances tensioactives qui présentent une valeur HLB supérieure à 35,
- 35 à 50% en poids d'un ou de plusieurs composants huileux,
- 0,2 à 5% en poids d'un ou de plusieurs polyacrylates, choisis dans le groupe formé par les homopolymères et/ou les copolymères anioniques de l'acide acrylique et/ou des dérivés alkylés de l'acide acrylique ainsi que leurs esters et
- 5 à 60% en poids d'eau.

2. Emulsions de nettoyage cosmétiques ou dermatologiques selon la revendication 1, **caractérisées en ce qu'**elles contiennent en outre d'autres adjuvants, additifs et/ou substances actives cosmétiques ou pharmaceutiques, qui diminuent la teneur en eau des formulations.

3. Emulsions de nettoyage cosmétiques ou dermatologiques selon l'une quelconque des revendications précédentes, **caractérisées en ce que** le ou les polyacrylates sont choisis dans le groupe des polymères de l'acide acrylique et/ou ses esters et/ou dans le groupe des copolymères d'acrylate-acrylate d'alkyle.

4. Emulsions de nettoyage cosmétiques ou dermatologiques selon l'une quelconque des revendications précédentes, **caractérisées en ce que** la substance tensioactive est le laurethsulfate de sodium.

5. Emulsions de nettoyage cosmétiques ou dermatologiques selon l'une quelconque des revendications précédentes, **caractérisées en ce qu'**elles contiennent, par rapport au poids total des compositions
- 5 à 20% en poids d'une ou de plusieurs substances tensioactives détergentes choisies dans le groupe des substances tensioactives qui présentent une valeur HLB supérieure à 35,
- 35 à 50% en poids d'un ou de plusieurs composants huileux,
- 0,5 à 2% en poids d'un ou de plusieurs polyacrylates, choisis dans le groupe formé par les homopolymères et/ou copolymères anioniques de l'acide acrylique et/ou des dérivés alkylés de l'acide acrylique ainsi que leurs esters et
- 5 à 60% en poids d'eau.
